# EUROPEAN PATENT APPLICATION

(11) **EP 2 072 504 A1**
(43) Date of publication of application: **24.06.2009**
(21) Application number: 07024744.0
(22) Date of filing: 20.12.2007
(51) Int. Cl.: C07D 251/62, B01J 8/02

(54) **Process for the preparation of melamine**

(71) Applicant: DSM IP Assets B.V., 6411 TE Heerlen (NL)
(72) Inventor: Groothaert, Marijke Hilde Leen, 3630 Maasmechelen (BE); Tjiou, Tjay Tjien, 135 JA Sittard (NL); Spoel, van der, Jan, 6132 HL Sittard (NL)
(74) Representative: van Tol-Koutstaal, Charlotte A.

(57) **Abstract**

The invention relates to a process for the preparation of melamine which process comprises at least the following steps:
a. feeding ammonia and hydrogen cyanide, either separately or combined, to a first reactor that contains a catalyst that comprises at least one group 11 element and which reactor is maintained at a pressure of at least 2 bar and a temperature of 300-500 °C, at a space velocity lying between 10² and 10⁶ ml/(g.hr), whereby a gaseous mixture (a) is formed comprising at least melamine,
b. optionally purifying mixture (a) producing a melamine containing mixture (b),
c. separating at least a part of the formed melamine thereby forming reaction mixture (c) and separated melamine

The invention also relates to a process for the preparation of melamine, which process comprises at least the following steps:
i. feeding methane and ammonia to a pre- reactor which pre- reactor is maintained at a temperature between 700 and 1500 °C whereby a gaseous mixture (i) is formed comprising at least hydrogen cyanide,
ii. purifying mixture (i) thereby forming a mixture (ii) that contains at least hydrogen cyanide,
a. feeding hydrogen cyanide from mixture (ii) and ammonia, either separately or combined, to a first reactor that contains a catalyst and which reactor is maintained at a pressure of at least 2 bar and a temperature of 300-500 °C, at a space velocity lying between 10² and 10⁶ ml/(g.hr), whereby a gaseous mixture (a) is formed comprising at least melamine,
b. optionally purifying mixture (a) producing a melamine containing mixture (b),
c. separating at least a part of the formed melamine thereby forming reaction mixture (c) and separated melamine

The invention further relates to the melamine obtained by the processes.

## Description

The invention relates to a process for the preparation of melamine starting from hydrogen cyanide and ammonia in the presence of a catalyst.

Such a process is known for example from GB-766.925, where cyanamide and/or melamine are produced from a vapor mixture containing hydrogen cyanide and oxygen over a catalyst at elevated temperatures of at least 350 °C. The catalyst in GB-766.925 is an inert adsorptive material having a surface area in the range of 180-650 m²/g. It is described there that ammonia can be present when desired. However, the obtained selectivities in GB-766.925 are rather low.

A further disadvantage of a melamine production process that makes use of an oxygen- containing reactant is that a larger range of by- products is possible, thus complicating purification of the melamine obtained in the process. Another drawback of the presence of oxygen is that water can be formed easily. The presence of water is detrimental as it might hydrolyze cyanamide that is a precursor to the formation of melamine. A further drawback of the use of oxygen-containing reactants is the loss of valuable feedstock via the formation of CO₂ and water.

Another type of process for the production of melamine starting from hydrogen cyanide and ammonia is described in GB-803.195. The described process runs at substantially atmospheric pressure in the presence of a dehydrogenation catalyst which comprises an oxide of chromium and/or a metal of Group VIII of the Periodic System. Examples given of the Group VIII metals are platinum, palladium, nickel and cobalt.

A disadvantage of a process such as described in GB-803.195 is the low yield that can maximally be obtained. Yields of the order of only 3% can be reached.

It is the object of the invention to reduce or even overcome the above-mentioned disadvantages. Therefore it is an object of the invention to provide a process for the production of melamine starting from hydrogen cyanide and ammonia with an increased yield and/or selectivity compared to the state of the art.

This object was reached by providing a process for the preparation of melamine which process comprises at least the following steps:
a. feeding ammonia and hydrogen cyanide, either separately or combined, to a first reactor that contains a catalyst that comprises at least one group 11 element and which reactor is maintained at a pressure of at least 2 bar and a temperature of 300-500 °C, at a space velocity lying between 10² and 10⁶ ml/(g.hr), whereby a gaseous mixture (a) is formed comprising at least melamine,
b. optionally purifying mixture (a) producing a melamine containing mixture (b),
c. separating at least a part of the formed melamine thereby forming reaction mixture (c) and separated melamine

In the process according to the invention ammonia and hydrogen cyanide are fed to the reactor by generally known means and apparatus. Ammonia and hydrogen cyanide can be combined before they are fed to the reactor, however they can also be fed separately to the reactor. The amount of ammonia and hydrogen cyanide in the feed stream to the reactor can come from new supply of the materials, however it is also possible to use at least a part of reaction mixture (c) and recycle this part of reaction mixture (c) to the reactor. When the recycle is used, the used ammonia and hydrogen cyanide should be replenished by a new supply of those materials.

The ammonia and hydrogen cyanide as feed to the reactor can be preheated before entering the reactor or they can be used as such. When recycled ammonia and hydrogen cyanide is used this can be used to pre- heat the feed stream to the reactor.

The pressure in the reactor is at least 2 bar or higher. It is preferred to use a pressure in the reactor of at least 5 bar, more preferably the pressure is at least 10 bar. It has been found that at high pressures the yield of melamine is much higher than at low pressures, such as for example ambient pressure. Here and hereinafter the pressure is used as the absolute pressure. The pressure in the reactor is preferably kept below 200 bar, more preferably below 100 bar. At very high pressures the construction of the reactor and other apparatus tends to be very heavy, increasing the investments costs considerably. Therefore it is preferred to use lower pressures. A suitable range for the pressure in the reactor is 2-200 bar. A preferred pressure in the reactor lies within the range of 5-100 bar, more preferably 5-50 bar.

The temperature in the reactor is maintained between 300 and 500 °C. Preferably the temperature is kept between 350 and 450 °C (borders included), because it has surprisingly been found that within this range the yield and selectivity to melamine are most favorable.

The reaction should be carried out at a space velocity lying between 10² and 10⁶ millilitre of reaction mixture per gram of catalyst per hour (ml/(g.h)) (borders included). It was found that the occurrence of undesired side -reactions was minimised at higher space velocities. The space velocity is preferably at least 3.10² ml/(g.h), more preferably at least 1.10³ ml/(g.h) and most preferably at least 3.10³ ml/(g.h). It was found that the melamine yield was maximised when the space velocity is at most 10⁶ ml/(g.h). Preferably the space velocity is below 3.10⁵ ml/(g.h), even more preferably the space velocity is below 10⁵ ml/(g.h)

The catalyst that is used in the process according to the invention comprises at least one group 11 element. With "group 11 element" is meant an element from group 11 of the Periodic Table of Elements. A current Internet reference for the IUPAC Periodic Table of Elements is www.iupac.orq/reports/periodic table; the version as used here and hereinafter is dated June 22, 2007. Within the context of the present invention, the term "group 11 element" is understood to mean the element itself, a compound comprising the element or a mixture of the element and/or compound comprising the element.

The catalyst will generally comprise a support material and at least one catalytic active element. The support material is used to increase the catalyst surface area and also for reasons of ease of handling and sturdiness. It is preferred to use the group 11 element supported on at least one heat resistant inorganic compound, more preferably the support is made from a heat resistant inorganic material with a high surface area.

Within the context of the present invention, the term 'support' is understood to mean one heat resistant inorganic compound or a mixture of two or more heat resistant inorganic compounds. Examples of suitable heat-resistant inorganic compounds are alumina, silicon carbide or other carbon-containing supports, silica, silica-alumina, titanium dioxide, silica magnesia, magnesium oxide, diatomaceous earth, zeolite, pumice, zirconium oxide, cerium oxide, calcium sulphate, titanium phosphate, silicon phosphate and mixtures of any of them. A preferred support material is chosen from the list comprising silica, alumina, silica-alumina, zeolite, titanium dioxide, magnesium oxide and mixtures of any of them.

When one group 11 element is present in the catalyst, the amount of it generally lies within the range 0.5-50 wt%. Preferably the amount of group 11 element lies within the range 5-30 wt%, more preferably 15-25 wt%. The preferred group 11 element is copper. Preferably copper is present in an amount of between 18 and 28 wt%.

When more than one group 11 element is present in the catalyst, the different group 11 elements can be present on or in the same support or on or in different supports. The amount of the active components, i.e. components showing catalytic activity, to the total weight of the catalyst varies, depending amongst others upon the support used, method of catalyst preparation and atom ratio of the active components, but is generally at least 0.1 and generally at most 99 wt%. Preferably the atom ratio is at least 1 wt% more preferably at least 2 wt% and most preferably at least 5 wt%. Preferably the atom ratio is at most 95 wt%, more preferably at most 90 wt%, even more preferably at most 80 wt% and most preferably at most 70 wt%.

Another preferred catalyst for use in the process according to the invention comprises next to the at least one group 11 element, at least one other element from the transition metals, the alkali metals and/or the lanthanide group. The transition metals here referred to are considered to be the elements in the groups 3-12 of the Periodic Table. The alkali metals are considered to be the elements in group 1 of the Periodic Table. The elements from the lanthanide group are considered to be the elements with atomic number 57 (Lanthanide) up to and including 71 (Lutetium).

When next to the group 11 element(s), at least one other of transition metal, alkali metal or element from the lanthanide group is present in the catalyst, they can be present on or in the same support or on or in different supports. The amount of these active components, i.e. components showing catalytic activity, to the total weight of the catalyst varies, depending amongst others upon the support used, method of catalyst preparation and atom ratio of the active components, but is generally at least 0.1 and generally at most 99 wt%. Preferably the atom ratio is at least 1 wt%, more preferably at least 2 wt% and most preferably at least 5 wt%. Preferably the atom ratio is at most 95 wt%, more preferably at most 90 wt%, even more preferably at most 80 wt% and most preferably at most 70 wt%.

Examples of suitable transition metals are titanium, vanadium, chromium, molybdenum, manganese, ruthenium, cobalt, nickel, palladium, platinum and silver. Preferred transition metals are vanadium, ruthenium, cobalt, nickel, palladium and silver, most preferred transition metals are vanadium, cobalt and silver. Examples of suitable elements from the alkali metals are lithium, potassium, rubidium and cesium, preferred elements are lithium and cesium, most preferred element from the alkali metals is lithium. Examples of suitable elements from the lanthanide group are lanthanum, cerium, praseodymium, ytterbium and lutetium. Preferred elements are cerium and praseodymium, most preferred element from the lanthanide group is praseodymium.

An especially preferred catalyst comprises next to the copper both another element from group 11, an other transition metal and/or an alkali metal and/or an element from the lanthanide group. A very advantageous catalyst was found to be a catalyst that comprises next to the support: copper, vanadium, silver, praseodymium, lithium and cobalt.

The catalyst for use in the process according to the invention preferably contains the group 11 element in an amount higher than the individual amounts of the components showing catalytic activity. The at least one group 11 element is thus preferably the major (by weight) component showing catalytic activity.

The catalyst to be used in the process according to the invention may be prepared by methods known as such to the skilled person. Reference can for example be made to the "Encyclopedia of Catalysis", John Wiley & Sons, ed. István Horváth, 2007.

It has been found advantageous to pre-treat the catalyst before the reactants are fed to the catalyst. A suitable method is to pre-treat the catalyst in a flow containing hydrogen at an elevated temperature during an extended period of time.

The catalyst as used in the process according to the invention can have various shapes, such as for example small particles, granules, wires or gauzes. If the catalyst comprises or consists essentially of particles- either as such or in agglomerated or sintered form- then it is preferred that the said particles are in size between 100 nm and 5 mm. The term size is defined herein as the average value of the largest and smallest dimension of a particle.

Since the catalyst is essentially in the solid phase and the reaction mixture is essentially in the gaseous or supercritical phase, it follows that the catalyzed reaction step in the process according to the invention falls into the category of heterogeneous catalytic reactions.

It has been found that it is advantageous to perform the process according to the invention in the absence of added oxygen. When no stream of oxygen-containing gas is fed to the reactor the selectivity is better, purification of the products is easier and the process is economically more advantageous as no valuable feedstock is lost in the conversion to unwanted, oxygen-containing, products. With "no stream of oxygen-containing gas is fed to the reactor" is meant that oxygen is not intentionally fed to the reactor, however low amounts of oxygen can, unintentionally, enter the reactor for example as contamination in the feed stream or by tiny leakages in the apparatus. These low amounts of oxygen will not negatively influence the reactions taking place. It is preferred to keep the level of oxygen in the reactor below 2 vol%, more preferably below 1 vol%, most preferably below 0.5 vol%. It is even more preferred to perform the process according to the invention in the absence of oxygen.

The process for the preparation of melamine according to the invention makes use of a feed of ammonia and hydrogen cyanide to the reactor. Hydrogen cyanide can be bought as such from an external source; however transportation brings a lot of (health) risks with it. Therefore it would be an advantage to be able to synthesize the hydrogen cyanide in-situ, at the same location as where it will be used in further synthesis.

A process for the preparation of hydrogen cyanide is for example known as the BMA (methane-ammonia process). In this process that requires temperatures above 1200 °C, methane and ammonia are heated in alumina tube bundles coated with platinum. Reference can for example be made to Ullmann's Encyclopedia of Industrial Chemisty, VCH Verlag, 1987, pg 162-163. After the hydrogen cyanide is synthesized, the purification and isolation is rather complicated and expensive.

It is an object of the invention to provide an integrated process for the preparation of melamine wherein the hydrogen cyanide need not be transported between chemical sites and which is cheaper than prior art processes.

This object has been reached by a process for the preparation of melamine, which process comprises at least the following steps:
i) feeding methane and ammonia to a pre- reactor which pre- reactor is maintained at a temperature between 700 and 1500 °C whereby a gaseous mixture (i) is formed comprising at least hydrogen cyanide,
ii) purifying mixture (i) thereby forming a mixture (ii) that contains at least gaseous hydrogen cyanide,
   a. feeding hydrogen cyanide from mixture (ii) and ammonia, either separately or combined, to a first reactor that contains a catalyst and which reactor is maintained at a pressure of at least 2 bar and a temperature of 300-500 °C, at a space velocity lying between 10² and 10⁶ ml/(g.hr), whereby a gaseous mixture (a) is formed comprising at least melamine,
   b. optionally purifying mixture (a) producing a melamine containing mixture (b),
   c. separating at least a part of the formed melamine thereby forming reaction mixture (c) and separated melamine

This process will hereinafter for ease of reference be referred to as the "methane-melamine process". The process for the preparation of melamine starting from hydrogen cyanide will be referred to as "hydrogen cyanide- melamine process".

In the methane-melamine process the temperature of the pre-reactor is generally between 700 and 1500 °C. A preferred range for the temperature is 900-1400 °C. The pressure in the pre-reactor is generally kept at moderate pressures. A suitable pressure range is 1-5 bar, preferably 1-3 bar. Generally a catalyst will be present. Suitable catalysts for this process step are known to the man skilled in the art. An example is a platinum based catalyst. Methane and ammonia in the feed are converted to hydrogen cyanide and one or more byproducts. The gaseous mixture (i) that is formed in step i) of the process comprises at least hydrogen cyanide and possibly next to that hydrogen gas, nitrogen, unreacted methane and/ or ammonia

In the process according to the invention the gaseous mixture (i) is purified so as to make it possible to use part of the constituents of the mixture in subsequent steps or to isolate the constituents for further use or disposal. After purifying mixture (i) a mixture (ii) is formed that contains at least gaseous hydrogen cyanide. Mixture (ii) is enriched in hydrogen cyanide compared to mixture (i). The hydrogen cyanide from mixture (ii) is now fed, either separately or combined with ammonia to a first reactor that contains a catalyst.

It has been found advantageous to add a cooling step after step (i), whereby reaction mixture (i) is cooled down to a temperature between 500-300 °C. Preferably the temperature to which is cooled down is between 450 and 350 °C. The advantage of this additional step is that it becomes possible to separate hydrogen that is produced in the hydrogen cyanide reaction, from the other constituents in the mixture. The separated hydrogen can now be used in all kinds of applications, such as for example in other synthesis routes or as fuel gas for a furnace. By separating the hydrogen and using it in other processes the overall costs for the production of melamine are reduced. Additionally it has been found that by removal of hydrogen the yield to melamine is increased.

It has been found advantageous to include in the process a compression step as an additional step after step i. whereby reaction mixture (i) is compressed to a pressure between 5 and 50 bar. This step can be used separately from or combined with the additional step of cooling down reaction mixture (i).

The catalyst that is present in the first reactor, in step a., can be any catalyst that is able to catalyze the reaction between ammonia and hydrogen cyanide. Suitable examples are chromium oxide catalysts, catalysts with an inert material in combination with a heavy metal oxide, quartz with phosphoric acid. A preferred catalyst however is a catalyst that comprises at least one group 11 element. A further preferred catalyst comprises next to the at least one group 11 element, at least one element from the transition metals, the alkali metals and/or the lanthanide group. For further details and preferences reference is made to what has been written in connection with the catalyst for the hydrogen cyanide- melamine process. The same preferences hold for the catalyst in the hydrogen cyanide- melamine process as for the catalyst in step a. in the first reactor of the methane- melamine process.

The present invention will now be elucidated by the following nonlimiting examples.

### EXAMPLES

### General experimental procedure

A reactor of diameter 1/4 inch was packed with a pelletized catalyst with typical sieve fraction of 0.5-1 mm. The solid catalyst bed was packed into the reactor so that it is centred at the part of the reactor which will be in the centre of the reaction oven. The catalyst bed was held in place with quartz wool bungs at either end, and the reactor below the catalyst bed was packed with SiC (0.25 mm fraction) to prevent downward movement of the catalyst. The SiC was held in the reactor with a quartz wool bung at the base of the reactor.

The reactor was built into the reaction setup and the catalyst bed was pre-treated with H₂ at a flow of 50 ml/(g.h) for about 12 hours at 500 °C. The oven temperature was ramped to 500 °C at 5 °C per minute and after pre-treatment ramped to reaction temperature.

Following pre-treatment, the gas flow was switched to helium to flush the H₂ out of the system. The system was pressurized using a back pressure valve.

The start of the reaction was marked by shutting the helium valve and opening the valves to the reaction gases (HCN and NH₃) simultaneously. Reaction gas flows were controlled using control boxes to regulate high pressure mass flow controllers. The reactor outlet flow composition was monitored by constant online MS and regular online GC sampling. Reaction duration was at least 4 hours.

At the end of the reaction duration, the reactant gases were switched off, and N₂ was flushed through the system while decreasing the temperature to room temperature and releasing pressure.

After opening the reactor system, the SiC and catalyst were subsequently removed from the reactor and separately extracted with water. Both samples were analysed by LC-UV on melamine concentration. Melamine yield (given in %) is defined as the amount of carbon as fed to the reactor in the form of HCN to have reacted into melamine.

### Preparation of the catalysts

### Catalyst 1:

A catalyst was prepared with the following composition, based on weight percentage:
Cu 23.0 - V 1.1 - Ag 0.3 - Pr 1.7 - Co 0.34 - Li 18.0 - balance SiO₂.

The materials used for the catalyst preparation were silica Aerosil type OX380 as the carrier of the catalyst and metal compounds of laboratory grade. 120 gram of the silica was, under continuous stirring, added to 1500 grams of distilled water so as to obtain a dispersed solution with the silica (dispersed solution I).

136.42 grams of copper nitrate trihydrate, 0.58 grams of silver nitrate and 2.04 grams of cobalt nitrate hexahydrate and 5.25 gram of praseodymium chloride hexahydrate were added to 1000 grams of distilled water. The pH was adjusted to 1.9 by dripping with concentrated nitric acid so as to obtain a homogeneous solution. This homogeneous metal solution was added to the dispersion with silica (dispersed solution I) under continuous stirring, resulting in a dispersed solution II.

First, 3.07 grams of ammonium meta- vanadate was added to 50 grams of distilled water so as to obtain a dispersed vanadate- solution. This dispersed vanadate- solution was added to the silica containing dispersion (II) under continuous stirring, resulting in a dispersion (IV). Finally, 28 grams of Lithium hydroxide was added to 100 ml of distilled water so as to obtain a homogeneous Li-solution and this Li-solution was added to the silica containing dispersion (IV) under continuous stirring.

This final solution was stirred for one hour and then filtrated over a paper filter with a Buchner funnel. First the wet cake was dried at 100 °C in a stove and then calcined at 310 °C during 16 hours. The resulting material was sieved and the fraction of 0.5 - 1.0 mm was collected. The BET surface of this material was 60 m²/g and the pore volume was 0.77 grams per ml.

### Comparative Catalyst 2:

A catalyst was prepared with the following composition, based on weight percentage:
Pd 0.5 - MgO.

MgO (Janssen Chimica 97%) was impregnated via incipient wetness impregnation with an aqueous solution of Pd(NO₃)₂ (Merck, 99%) in deionised water. The catalyst was dried in an oven at 80 °C and then calcined in flowing air (10nL/h), ramped (2°C/min) to 350 °C and kept at this temperature for 2 hours.

### Example 1

The plug flow reactor was filled with 0.24 g of the pelletized Cu-V-Ag-Pr-Co-Li/SiO₂ catalyst as prepared above (catalyst 1). After catalyst pre-treatment in H₂, cooling down to the desired reaction temperature of 400°C and pressurizing to 50 bar, the reactant gases were led through the reactor with a total gas hourly space velocity of 2.10⁴ ml/(g.h). The inlet gas flow composition amounted to 0.1 vol% HCN, 36.5 vol% NH₃ and 63.4 vol% He. Of the amount of carbon as fed to the reactor in the form of HCN, 4.7 % was found to have reacted into melamine. Of all melamine formed, more than 99% was found on the SiC, i.e. the cooler part below the catalyst and less than 1% was extracted from the catalyst. Selectivity to melamine was 98%.

### Example 2

The general procedure as described above was followed, except that the reactor was packed with the double amount of catalyst, reducing the gas hourly space velocity to 1.10⁴ ml/(g.h). In this experiment a melamine yield of 12.3 % was obtained.

### Example 3

The same settings were applied as in Example 2, except that the reactor was operated at the lower pressure of 40 bar. In this experiment a melamine yield of 10.6 % was obtained.

### Example 4

The same settings were applied as in example 2, except that the reactor was operated at the lower temperature of 380 °C. In this experiment a melamine yield of 4.6 % was obtained.

### Comparative Experiment I

The same settings were applied as in Example 3, except that no catalyst was packed in the reactor. In this experiment a melamine yield of only 0.13% was obtained.

### Comparative Experiment II

The same settings were applied as in Example 1, except that the reactor was packed with the Comparative catalyst 2: Pd-MgO catalyst. In this experiment a melamine yield of only 0.32% was obtained.

### Comparative Experiment III

The same settings were applied as in Example 1, except that the reactor was operated at atmospheric pressure. In this experiment a melamine yield of only 0.18% was obtained

## Claims

1. Process for the preparation of melamine which process comprises at least the following steps:
a. feeding ammonia and hydrogen cyanide, either separately or combined, to a first reactor that contains a catalyst that comprises at least one group 11 element and which reactor is maintained at a pressure of at least 2 bar and a temperature of 300-500 °C, at a space velocity lying between 10² and 10⁶ ml/(g.hr), whereby a gaseous mixture (a) is formed comprising at least melamine,
b. optionally purifying mixture (a) producing a melamine containing mixture (b),
c. separating at least a part of the formed melamine thereby forming reaction mixture (c) and separated melamine

2. Process according to claim 1 **characterized in that** the at least one group 11 element is supported on at least one heat resistant inorganic compound

3. Process according to claim 1 or 2 **characterized in that** the group 11 element is copper

4. Process according to claim 2 or 3 **characterized in that** the support material is chosen from the list comprising silica, alumina, silica-alumina, zeolite, titanium dioxide, magnesium oxide and mixtures of any of them

5. Process according to anyone of claim 1-4 **characterized in that** the catalyst comprises next to the group 11 element at least one other element from the transition metals, alkali metals and/ or the lanthanide group

6. Process according to anyone of claim 1-5 **characterized in that** the catalyst comprises next to the support, copper, vanadium, silver, praseodymium, lithium and cobalt

7. Process according to anyone of claim 1-6 **characterized in that** the group 11 element is present in an amount higher than the individual amounts of the components showing catalytic activity.

8. Process according to anyone of claim 1-7 **characterized in that** the reactor is maintained at a pressure of at least 5 bar

9. Process according to anyone of claim 1-8 **characterized in that** the reactor is maintained at a temperature of 350-450 °C

10. Process for the preparation of melamine, which process comprises at least the following steps:
i) feeding methane and ammonia to a pre- reactor which pre- reactor is maintained at a temperature between 700 and 1500 °C whereby a gaseous mixture (i) is formed comprising at least hydrogen cyanide,
ii) purifying mixture (i) thereby forming a mixture (ii) that contains at least gaseous hydrogen cyanide,
a. feeding hydrogen cyanide from mixture (ii) and ammonia, either separately or combined, to a first reactor that contains a catalyst and which reactor is maintained at a pressure of at least 2 bar and a temperature of 300-500 °C, at a space velocity lying between 10² and 10⁶ ml/(g.hr), whereby a gaseous mixture (a) is formed comprising at least melamine,
b. optionally purifying mixture (a) producing a melamine containing mixture (b),
c. separating at least a part of the formed melamine thereby forming reaction mixture (c) and separated melamine

11. Process for the preparation of melamine according to claim 10 comprising a cooling step as an additional step after step i. whereby reaction mixture (i) is cooled down to a temperature between 500-300 °C.

12. Process for the preparation of melamine according to claim 10 or 11 comprising a compression step as an additional step after step i. whereby reaction mixture (i) is compressed to a pressure between 5 and 50 bar

13. Process for the preparation of melamine according to anyone of claim 1-12 wherein reaction mixture (c) is either recycled to the reactor in step a. or to the pre- reactor in step i.

14. Melamine obtainable by the process according to anyone of claim 1-13
